# EUROPEAN PATENT SPECIFICATION

(11) **EP 1 728 531 B1**
(45) Date of publication and mention of the grant of the patent: **09.12.2009**
(21) Application number: 06011369.3
(22) Date of filing: 01.06.2006
(51) Int. Cl.: A61M 25/00, A61B 17/28

(54) **Thrombus suction catheter**
Thrombusabsaugkatheter
Cathéter d'aspiration pour aspirer un thrombus

(30) Priority: 01.06.2005 JP 2005162005
(43) Date of publication of application: 06.12.2006
(73) Proprietor: Nipro Corporation, Osaka-shi, Osaka 531-8510 (JP)
(72) Inventor: Mori, Satoru, Kita-ku, Osaka-shi Osaka 531-8510 (JP)
(74) Representative: Banzer, Hans-Jörg

(56) References cited:
- EP-A- 1 440 663
- EP-A- 1 639 951
- US-A- 5 938 645

## Description

### FIELD OF THE INVENTION

This invention relates to a thrombus suction catheter which is introduced into the body through the skin and sucks and removes debris (foreign matter) such as thrombus formed in blood vessels and atheroma liberated in blood vessels out of the body relying on a negative pressure applied through the proximal end of the catheter.

### BACKGROUND OF THE INVENTION

Thrombic diseases are not only those of cardiac (coronary arteries) diseases as represented by acute cardiac infarction but are also those occurring in the blood vessels of hands and legs. So far, these diseases were cured by surgical therapy which, however, has been replaced by an intervention therapy (catheter therapy through the skin) which is a low invasive therapy that is now rapidly spreading. This is because low invasive therapy is preferably carried out over therapies of a high invasive degree from the standpoint of quality of life (QOL) of the patient who is to be treated.

This invention relates to a thrombus suction therapy which is one of the intervention therapies. Thrombus suction therapy is one according to which a fine tube called a catheter is inserted up to a portion to be treated to suck and remove thrombus built up in the limbs such as the legs and arms.

The catheter for removing the thrombus is called a thrombus suction catheter and is used in combination with a suction tool. The thrombus suction catheter must have a strength such that the catheter wall will not be crushed by negative pressure and must further have an area (opening area) of the opening which is as large as possible in cross section, so that the sucked material can be easily discharged out of the body from the diseased part.

In conventional catheters, it has been attempted to improve the suction performance by using a single lumen tube. Without using a guide wire lumen for holding the guide wire, however, a difference between the inner diameter of the catheter and the outer diameter of the guide wire makes it difficult to operate the catheter in a desired direction. Furthermore, with the catheter of the so-called rapid exchange type that is generally used for the heart, the guide wire is located on the outer side of the catheter relative to the inner diameter of the sheath introducer which advances the catheter. Therefore, the size of the catheter itself must be decreased by an amount of the outer diameter of the guide wire so as to be smaller than the inner diameter of the sheath introducer, causing such a defect that the sectional area of the suction lumen must be decreased. Such conventional catheters are disclosed in U.S. Patent No. 5,827,229 (Fig. 5(a), Fig. 6(d)), Japanese Patent No. 2812713 or JP-A-2004-65326.

From US 5,938,643 a thrombus section catheter according to the preamble of claim 1 is known which is formed as a single lumen catheter 18 with a tip having a slanted thrombus entry opening. In an example, distal from the opening a portion is provided forming a lumen for a guide wire which does not communicate with a suction lumen. In another example, an opening is provided to allow a guide wire to run within a single lumen of the catheter.

A similar catheter is known from EP 1 639 951 A1.

From EP 1 440 663 a thrombus section catheter is known comprising a tube having a distal end opening formed by an angled cut surface. A second lumen is provided for a guide wire, said second lumen extending from an insertion port to the distal end opening.

### SUMMARY OF THE INVENTION

It is therefore an object of the present invention to improve the sucking force of a thrombus suction catheter by increasing the sectional area of the suction lumen and the operability of the catheter, which are conflicting functions with the conventional catheters.

This object is achieved by a catheter according to claim 1. The dependent claims define further embodiments of the catheter.

In order to solve the above problems, the present inventors have conducted a keen study and have arrived at a structure in which a distal end portion of a catheter is provided with a short guide wire shaft, the opening at the distal-most end portion of the catheter is formed by an angled cut surface to accomplish better permeation into the blood vessel and into the diseased part, and in which only a guide wire is permitted to advance into the tubular body to minimize an undesired decrease in the sectional area of the suction lumen.

That is, this invention is concerned with a thrombus suction catheter including a tubular body which has a suction lumen penetrating from a proximal end to a distal end thereof, and an opening formed by an angled cut surface angled with respect to the axis of the catheter at a distal end portion thereof, wherein a guide wire lumen is formed as a separate shaft in a guide wire shaft provided in a neck-down tip extending from the opening of the tubular body only in the distal most end portion of the tubular body at the distal end, the guide wire lumen communicating with the suction lumen.

In a preferred embodiment, the cut surface terminating in a necked-down tip at a distal side of the cut surface, and at least a portion of the cut surface on a proximal side of the cut surface being concave in the angled direction.

Further, the distal end portion of the tubular body comprises a resin kneaded with a contrast material for confirming the position of the catheter by X-ray examination or has an X-ray impermeable marker. The tubular body has a metal-knitted or coil reinforcing member. Further, the tubular body is provided with a hydrophilic coating. Additionally, the tubular body has differences in hardness in a plurality of steps which depend upon the differences in the hardness of the resins in a manner of becoming softer toward the distal end.
Here, the distal end portion of the tubular body comprises a resin kneaded with a contrast material for confirming the position of the catheter in an X-ray examination or has an X-ray impermeable marker.

### BRIEF DESCRIPTION OF THE DRAWINGS

Fig. 1 is a front view illustrating the entire thrombus suction catheter according to an embodiment of the present invention.
Fig. 2 is a sectional view illustrating a distal end portion M of the catheter of Fig. 1 on an enlarged scale.
Fig. 3 is a plan view illustrating a major distal end portion M of the catheter of Fig. 1 on an enlarged scale.
Fig. 4 is a sectional view of the catheter of Fig. 1 taken along A-A.
Fig. 5 is a sectional view of the catheter of Fig. 1 taken along B-B.
Fig. 6 is a sectional view of the catheter of Fig. 1 taken along C-C.

### PREFERRED EMBODIMENTS OF THE INVENTION

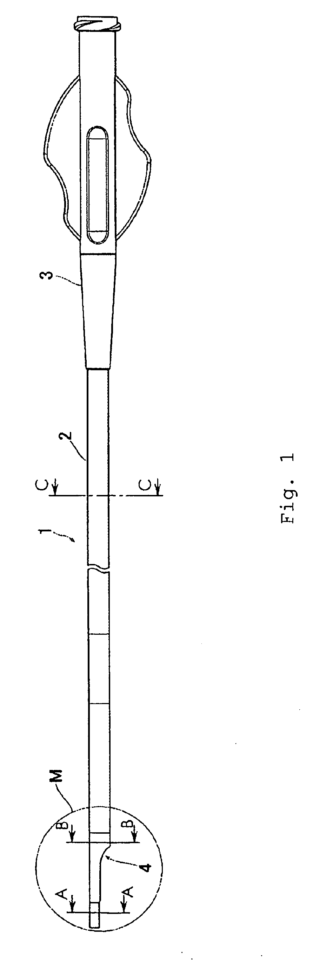

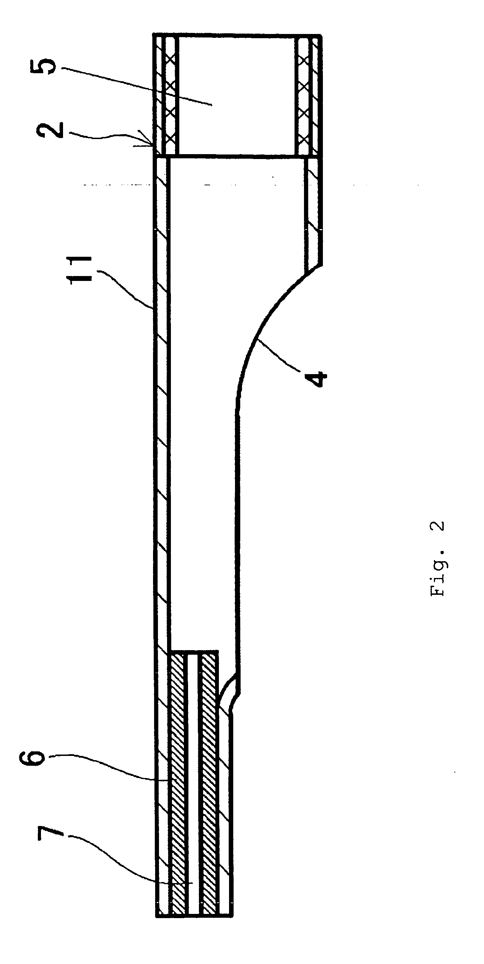

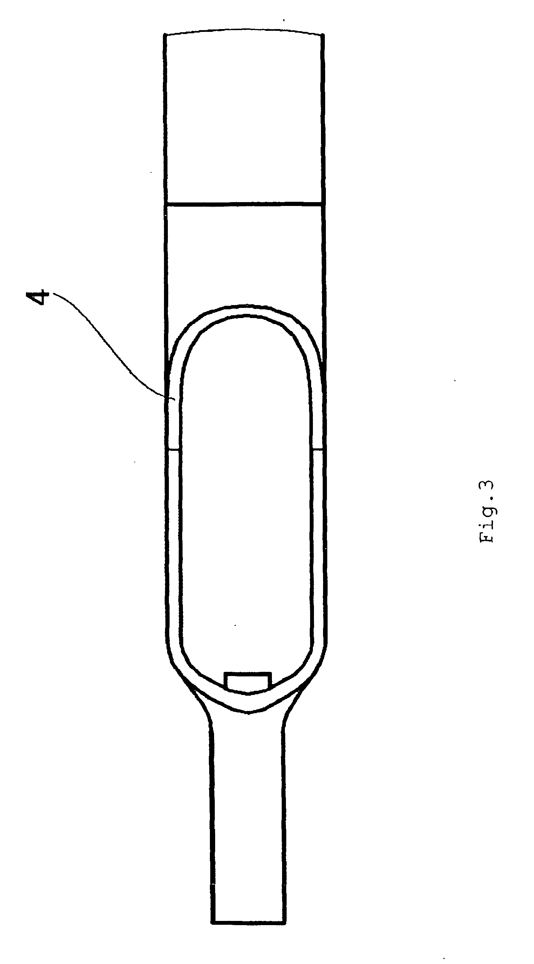

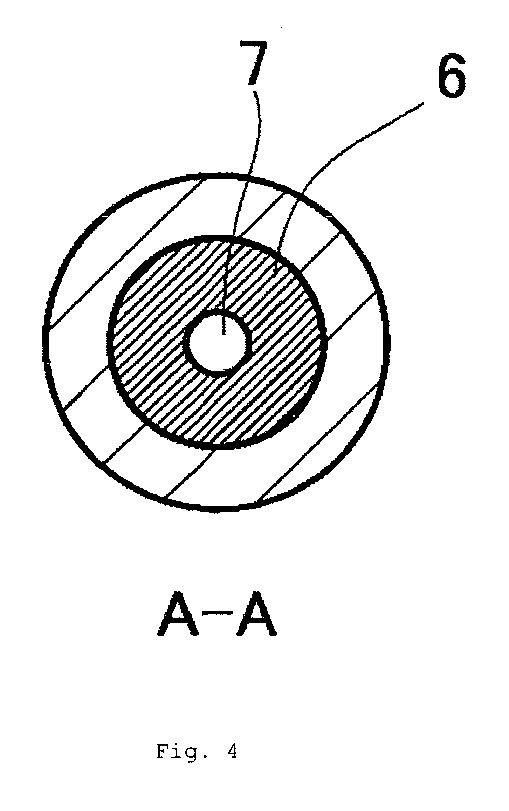

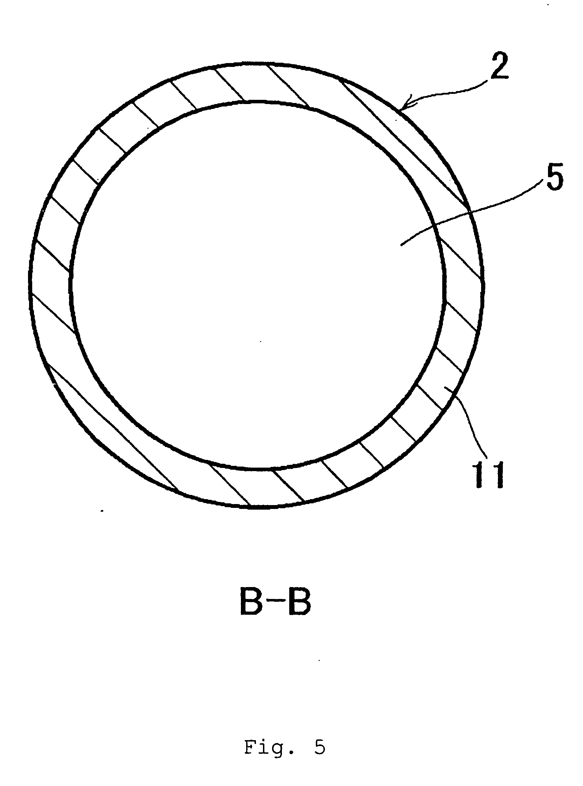

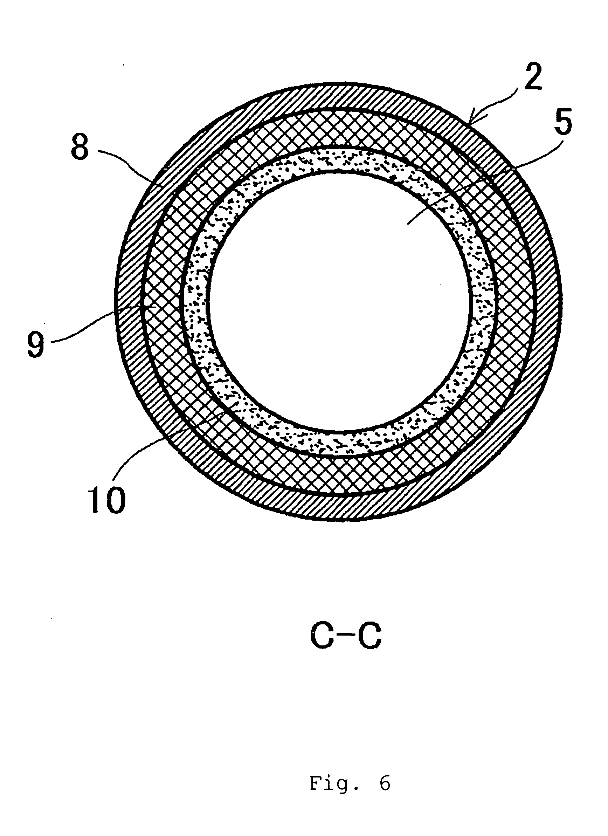

Described below is an embodiment of the thrombus suction catheter according to the present invention to which, however, the invention is in no way limited. The embodiment will be described hereinafter with reference to the drawings. Fig. 1 is a front view illustrating the entirety of the thrombus suction catheter according to an embodiment of the present invention, Fig. 2 is a sectional view illustrating a distal end portion M of the catheter of Fig. 1 on an enlarged scale, Fig. 3 is a plan view illustrating a major distal end portion M of the catheter of Fig. 1 on an enlarged scale, Fig. 4 is a sectional view of the catheter of Fig. 1 along line A-A, Fig. 5 is a sectional view of the catheter of Fig. 1 along line B-B, and Fig. 6 is a sectional view of the catheter of Fig. 1 along line C-C.

The thrombus suction catheter 1 according to the present invention includes a tubular body 2 which has a suction lumen 5 penetrating from a proximal end to a distal end thereof, an opening formed by an angled cut surface 4 at a distal end 11 thereof, the proximal end including a hub 3, the distal end portion 11 having a guide wire shaft 6 that follows the guide wire. The guide wire that passes through the suction lumen 5 communicates with a guide wire lumen 7 in the guide wire shaft 6 positioned at the most distal end portion.

The tubular body 2 has a guide wire lumen 7 at the distal end portion 11, and is of the so-called over-the-wire structure in which the guide wire that passes through the suction lumen 5 is communicates with the guide wire lumen 7. The operator advances the guide wire in the blood vessel up to a peripheral diseased part, so that the thrombus suction catheter 1 of the invention is delivered along the guide wire. Further, the angled cut surface 4 is formed at the distal end portion 11. Therefore, the distal end portion becomes soft and thinner than the proximal end side of the tubular body 2 making it possible to improve the operability/permeability in the blood vessel and to treat highly bent portions and branched portions.

In the catheter of the invention, the guide wire lumen has a length in the range of 3 to 15 mm and a diameter in the range of 0.46 to 1.00 mm. The outer diameter of the guide wire shaft is in the range of 0.86 to 1.40 mm. The tubular body of the catheter has an inner diameter in the range of 1.35 to 1.95 mm and an outer diameter in the range of 1.70 to 2.30 mm. Typical guide wires have a diameter of 0.36 mm, 0.46 mm or 0.89 mm.

The method of joining the guide wire shaft 6 to the distal end portion 11 of the tubular body 2 does not impose any limitation on the effect of the present invention. That is, the junction may be accomplished relying on a known method such as heat-melting or adhesion.

On the distal end side of the tubular body 2, there can be used a resin kneaded with a contrast material in the distal end portion 11 of the tubular body 2 or in the whole tubular body 2 for confirming the position of the distal end portion of the thrombus suction catheter 1 by an X-ray examination. The contrast material is obtained by blending a resin with a contrast material such as bismuth oxide, bismuth sulfate or bismuth subcarbonate in an amount of about 20% to about 60%.

Further, the guide wire shaft 6 at the distal end portion 11 can be provided with an X-ray impermeable marker. The material of the X-ray impermeable marker is desirably a single metal such as a stainless steel, gold, platinum or iridium, or a metal material such as an alloy.

A portion between the distal end 11 and the proximal end of the tubular body 2 is reinforced with a knit or a coil of metal mesh as a reinforcing body 9 in the tubular body 2 to resist the crush of the catheter wall surface caused by a negative pressure at the time of suction and to impart force against the pushing of the catheter. This increases the strength of the tube and expands the suction lumen 5 by decreasing the thickness of the catheter wall owing to the increased strength. As the material of the reinforcing member 9, there can be preferably used a metal material such as a stainless steel or Ti-Ni.

The tubular body 2 is further provided with differences in hardness in three or more steps based on the differences in the hardness of the resins in a manner of becoming softer toward the distal end and harder toward the proximal end.

At the diseased part in the blood vessel, therefore, a low hardness is provided while suppressing the permeability and invasion against the blood vessel. At the proximal end, no kinking occurs when operating or pushing the catheter which, therefore, can be pushed and turned.

Preferred structures include those structures/machining methods that have been used for contrast catheters/guiding catheters which are conventionally known. Though this is not to limit the effect of the present invention, preferred examples of the material constitution include three-layer structures as shown in Fig. 6, the inner layer 10 is formed using polyamide, polyamide elastomer, polyurethane, polyurethane elastomer, polyolefin, polyolefin elastomer, polyester, polyester elastomer or a fluorine-containing resin such as PTFE, FEP, ETFE or PFA, the intermediate layer 9 is a reinforcing member, and the outer layer 8 is polyamide, polyamide elastomer, polyurethane, polyurethane elastomer, polyolefin, polyolefin elastomer, polyester or polyester elastomer, and two-layer structure in which the inner layer is a reinforcing member and outer layer is as described above.

It is desired that the portion from the distal end to the proximal end of the tubular body 2 is provided with a hydrophilic coating from the standpoint of decreasing the invasion of the catheter against the blood vessel wall and improving permeability/operability.

The method of hydrophilic coating and the material are not intended to limit the effect of the invention, and can be selected depending upon the properties of the tubular body 2 and the guide wire shaft 6 that are used. For example, there can be preferably used a hydrophilic polymer such as a poly(2-hydroxyethyl methacrylate), a polyacrylamide or a polyvinyl pyrrolidone.

### Example 1

An example of the thrombus suction catheter of the invention will be described below.

The tubular body 2 has an outer diameter of 2.0 mm, an inner diameter of 1.65 mm, a length of 700 mm, and has a structure of an inner layer 10 of PFA and an intermediate layer 9 a reinforcing member of a stainless steel plain knitted structure, and an outer layer 8 of a nylon elastomer having a difference in hardness in three steps (resin hardnesses of 40D, 55D and 72D) along the length of the catheter.

A nylon elastomer blended with 40% of bismuth oxide is used as the contrast material of the distal end portion. The distal end portion 11 of the tubular body 2 is cut at angle by using a cutting blade to form an angled cut surface 4 extending to the distal end. A mandrel is inserted in a guide wire shaft 6 which has an outer diameter of 0.70 mm, an inner diameter of 0.55 mm and a length of 10 mm, the cut portion at the distal end portion 11 is wrapped around the guide wire shaft 6 and is melt-adhered by heat treatment, the mandrel is withdrawn and the distal end portion is trimmed.

A hub 3 injection-molded by using a polycarbonate is adhered to the proximal end of the tubular body 2 by using a UV adhesive, and a portion over a length of 200 mm beginning from the distal end of the catheter is provided with a hydrophilic coating thereby to obtain a thrombus suction catheter 1.

In the above embodiment, the guide wire lumen 7 is provided in the guide wire shaft 6. In an illustrative example not falling within the scope of the invention, the guide wire lumen 7 can be formed by simply wrapping the cut portion at the distal-most end of the distal end portion 11 around a mandrel or the like to form a lumen and heat-treating the wrapped portion.
In the above embodiment, it is desired that the tubular body 2 is flexible and has a difference in hardness in three or more steps in a manner of becoming harder toward the proximal end side. The difference in the hardness, however, may be in two steps.

### INDUSTRIAL APPLICABILITY

According to the present invention, the thrombus suction catheter is defined in claim 1. Therefore, sucking force is improved by the increased sectional area of the suction lumen and the operability of the catheter is improved, which are conflicting functions with the conventional catheters.

Further, the distal end portion of the tubular body comprises a resin kneaded with a contrast material for confirming the position of the catheter during X-ray examination or has an X-ray impermeable marker, offering a better view during therapy. The tubular body, further, has a metal-knitted or coil reinforcing member, improving the strength of the tube while decreasing the thickness thereof. Further, the tubular body can be provided with a hydrophilic coating, decreasing the invasion of the catheter against blood vessel walls and improving permeability/operability.

Additionally, the tubular body has differences in the hardness in three steps depending upon differences in the hardness of the resins in a manner of becoming softer toward the distal end. At a diseased part in a blood vessel, therefore, low hardness is provided while suppressing the permeability and invasion of the catheter against the blood vessel. At the proximal end portion, no kinking occurs when operating or pushing the catheter which, therefore, can be pushed and turned.

## Claims

1. A thrombus suction catheter (1) comprising a tubular body (2) having a suction lumen (5) penetrating from a proximal end to a distal end of the tubular body (2) and an opening for the suction lumen (5) formed by an angled cut surface (4) angled with respect to the axis of the catheter (1) at a distal end portion (11) of the tubular body (2), whereby
a separate guide wire lumen (7) is provided in a neck-down tip extending from the opening of the tubular body (2) only in a distal most end portion of said tubular body (2),
wherein said guide wire lumen (7) communicates with said suction lumen (5),
**characterized in that** the separate guide wire lumen (7) is formed as a separate shaft in a guide wire shaft (6).

2. The thrombus suction catheter (1) according to claim 1, wherein at least a portion of the angled cut surface (4) on a proximal side of said opening (51) is concave in the angled direction, and said angled cut surface (4) terminates in the necked-down tip at a distal side of said angled cut surface (4).

3. The thrombus suction catheter (1) according to claim 1 or 2, wherein the distal end portion (11) of said tubular body (2) comprises a resin kneaded with a contrast material or comprises an X-ray impermeable marker for confirming the position of the catheter in an X-ray examination.

4. The thrombus suction catheter (1) according to any one of claims 1 to 3, wherein said tubular body (2) includes a metal-knitted or coil reinforcing member.

5. The thrombus suction catheter (1) according to any one of claims 1 to 4, wherein said tubular body (2) is provided with a hydrophilic coating on an outer surface thereof.

6. The thrombus suction catheter (1) according to any one of claims 1 to 5, wherein said tubular body (2) has differences in hardness in a plurality of steps along the length thereof in a manner of becoming softer toward the distal end portion (11).

7. The thrombus suction catheter (1) according to any one of claims 1 to 6, wherein said distal most end portion is located at a distal end of said distal end portion (11).

8. The thrombus suction catheter (1) according to any one of claims 1-7, wherein the tubular body (2) has an inner diameter in the range of 1.35 mm to 1.95 mm and an outer diameter in the range 1.70mm to 2.30 mm, the guide wire shaft (6) has an outer diameter in the range of 0.86 mm to 1.40 mm, and the guide wire lumen has a diameter in the range of 0.46 mm to 1.00 mm.

## Patentansprüche

1. Thrombus-Absaugkatheter (1), umfassend einen Schlauchkörper (2) mit einem Sauglumen (5), das von dem proximalen Ende bis zum distalen Ende des Schlauchkörpers (2) verläuft, und eine Öffnung für das Sauglumen (5), gebildet durch eine winkelige Schnittfläche (4), die bezüglich der Achse des Katheters (1) abgewinkelt ist, am distalen Endabschnitt (11) des Schlauchkörpers (2), wobei ein getrenntes Führungsdrahtlumen (7) in einer querschnittsverengten Spitze vorgesehen ist, das sich von der Öffnung des Schlauchkörpers (2) nur in einen distalen äußersten Endabschnitt des Schlauchkörpers (2) erstreckt,
wobei das Führungsdrahtlumen (7) mit dem Sauglumen (5) in Verbindung steht,
**dadurch gekennzeichnet, dass** das getrennte Führungsdrahtlumen (7) als ein getrennter Schaft in einem Führungsdrahtschaft (6) ausgebildet ist.

2. Thrombus-Absaugkatheter (1) gemäß Anspruch 1, wobei wenigstens ein Abschnitt der abgewinkelten Schnittfläche (4) an einer proximalen Seite der Öffnung (51) in der abgewinkelten Richtung konkav ist und die abgewinkelte Schnittfläche (4) in der querschnittsverengten Spitze an der distalen Seite der abgewinkelten Schnittfläche (4) endet.

3. Thrombus-Absaugkatheter (1) gemäß Anspruch 1 oder 2, wobei der distale Endabschnitt (11) des Schlauchkörpers (2) ein Harz, das mit einem Kontrastmaterial verknetet ist, umfasst oder einen für Röntgenstrahlen undurchlässigen Marker umfasst, um die Position des Katheters bei einer Röntgenuntersuchung zu bestätigen.

4. Thrombus-Absaugkatheter (1) gemäß einem der Ansprüche 1 bis 3, wobei der Schlauchkörper (2) ein Metallgestrick- oder Spiralverstärkungselement umfasst.

5. Thrombus-Absaugkatheter (1) gemäß einem der Ansprüche 1 bis 4, wobei der Schlauchkörper (2) mit einer hydrophilen Beschichtung an einer Außenoberfläche desselben versehen ist.

6. Thrombus-Absaugkatheter (1) gemäß einem der Ansprüche 1 bis 5, wobei der Schlauchkörper (2) Unterschiede bei der Härte in einer Vielzahl von Schritten entlang der Länge desselben in einer Art hat, dass er in Richtung des distalen Endabschnitts (11) weicher wird.

7. Thrombus-Absaugkatheter (1) gemäß einem der Ansprüche 1 bis 6, wobei der distale äußerste Endabschnitt am distalen Ende des distalen Endabschnitts (11) lokalisiert ist.

8. Thrombus-Absaugkatheter (1) gemäß einem der Ansprüche 1 bis 7, wobei der Schlauchkörper (2) einen Innendurchmesser im Bereich von 1,35 mm bis 1,95 mm und einen Außendurchmesser im Bereich von 1,70 mm bis 2,30 mm hat, der Führungsdrahtschaft (6) einen Außendurchmesser im Bereich von 0,86 mm bis 1,40 mm hat und das Führungsdrahtlumen einen Durchmesser im Bereich von 0,46 mm bis 1,0 0mm hat.

## Revendications

1. Cathéter d'aspiration de thrombus (1) comprenant un corps tubulaire (2) ayant une lumière d'aspiration (5) pénétrant d'une extrémité proximale à une extrémité distale du corps tubulaire (2) et une ouverture pour la lumière d'aspiration (5) formée par une surface coupée angulaire (4) coudée par rapport à l'axe du cathéter (1) au niveau d'une partie d'extrémité distale (11) du corps tubulaire (2), moyennant quoi une lumière de fil guide séparée (7) est prévue dans une pointe étranglée s'étendant à partir de l'ouverture du corps tubulaire (2) uniquement dans la partie d'extrémité la plus distale dudit corps tubulaire (2),
dans lequel ladite lumière de fil guide (7) communique avec ladite lumière d'aspiration (5),
**caractérisé en ce que** la lumière de fil guide séparée (7) est formée comme une tige séparée dans une tige de fil guide (6).

2. Cathéter d'aspiration de thrombus (1) selon la revendication 1, dans lequel au moins une partie de la surface coupée angulaire (4) sur un côté proximal de ladite ouverture (51) est concave dans la direction coudée, et ladite surface coupée angulaire (4) se termine par la pointe étranglée au niveau d'un côté distal de ladite surface coupée angulaire (4).

3. Cathéter d'aspiration de thrombus (1) selon la revendication 1 ou 2, dans lequel la partie d'extrémité distale (11) dudit corps tubulaire (2) comprend une résine malaxée avec un matériau de contraste ou bien comprend un marqueur imperméable aux rayons X pour confirmer la position du cathéter lors d'un examen radiographique.

4. Cathéter d'aspiration de thrombus (1) selon l'une quelconque des revendications 1 à 3, dans lequel ledit corps tubulaire (2) comprend un élément tricoté en métal ou de renforcement hélicoïdal.

5. Cathéter d'aspiration de thrombus (1) selon l'une quelconque des revendications 1 à 4, dans lequel ledit corps tubulaire (2) est prévu avec un revêtement hydrophile sur sa surface externe.

6. Cathéter d'aspiration de thrombus (1) selon l'une quelconque des revendications 1 à 5, dans lequel ledit corps tubulaire (2) a des différences de dureté dans une pluralité d'étapes le long de sa longueur afin de devenir plus souple vers la partie d'extrémité distale (11).

7. Cathéter d'aspiration de thrombus (1) selon l'une quelconque des revendications 1 à 6, dans lequel ladite partie d'extrémité la plus distale est située au niveau d'une extrémité distale de ladite partie d'extrémité distale (11).

8. Cathéter d'aspiration de thrombus (1) selon l'une quelconque des revendications 1 à 7, dans lequel le corps tubulaire (2) a un diamètre interne de l'ordre de 1,35 mm à 1,95 mm et un diamètre externe de l'ordre de 1,70 mm à 2,30 mm, la tige de fil guide (6) a un diamètre externe de l'ordre de 0,86 mm à 1,40 mm et la lumière de fil guide a un diamètre de l'ordre de 0,46 mm à 1,00 mm.
